# EUROPÄISCHE PATENTSCHRIFT

(11) **EP 0 909 951 B1**
(45) Veröffentlichungstag und Bekanntmachung des Hinweises auf die Patenterteilung: **28.02.2001**
(21) Anmeldenummer: 97117290.3
(22) Anmeldetag: 07.10.1997
(51) Int. Cl.: G01N 33/00

(54) **Verfahren und Vorrichtung zur quantitativen Bestimmung des Gehalts von reinem und/oder gebundenem Wasserstoff**
Method and device for determining the amount of free and/or bound hydrogen
Méthode et dispositif pour la détermination quantitiv de hydrogène lié et/ou libre

(43) Veröffentlichungstag der Anmeldung: 21.04.1999
(73) Patentinhaber: Ipsen International GmbH, 47533 Kleve (DE)
(72) Erfinder: Feichtinger, Heinrich K., Professor Dr., 8128 Hinteregg (CH)
(74) Vertreter: Stenger, Watzke & Ring Patentanwälte

(56) Entgegenhaltungen:
- US-A- 4 882 032
- US-A- 5 088 315
- US-A- 5 668 301

## Beschreibung

Die Erfindung betrifft ein Verfahren zur quantitativen Bestimmung des Gehalts von reinem und/oder gebundenem Wasserstoff in wasserstoffhaltigen Gasgemischen, vorzugsweise in ammoniak- oder kohlenwasserstoffhaltigen Gasatmosphären von Wärmebehandlungsöfen. Die Erfindung bezieht sich ferner auf eine Vorrichtung zur Durchführung das Verfahrens.

Wasserstoff stellt eine bei vielen technischen Prozessen häufig anzutreffende Komponente eines Reaktionssystems mit wasserstoffhaltigen gasförmigen Komponenten dar. Derartige Reaktionssysteme sind beispielsweise die Reaktionen des Wasserstoffs mit den Spaltprodukten von Kohlenwasserstoffen oder Ammoniak, nämlich Kohlenstoff und Stickstoff, die bei zahlreichen thermochemischen Wärmebehandlungsverfahren von metallischen Werkstücken vorkommen. So wird zum Beispiel beim Gasnitrieren die Randschicht metallischer Werkstücke mit Stickstoff angereichert, um dadurch die Oberflächeneigenschaften, das heißt vor allem Härte, Verschleißwiderstand, Dauerfestigkeit oder Korrosionsbeständigkeit, zu verbessern. Als Stickstoff abgebendes Medium wird Ammoniak verwendet, dessen abgeschiedener Stickstoff unter Wasserstoff-Freisetzung zusammen mit stickstoffaffinen Legierungselementen der metallischen Werkstücke, wie beispielsweise Chrom, Aluminium, Titan, Vanadium etc., Nitride - oder bei gleichzeitiger Anwesenheit von Kohlenstoff Carbonitride - bildet, welche die gewünschten Werkstückeigenschaften hervorrufen.

Für die Charakterisierung derartiger Reaktionssysteme werden Kennzahlen herangezogen, die den Gehalt von Wasserstoff in ein Verhältnis zu dem Gehalt einer wasserstoffhaltigen Verbindung setzten. Eine solche Kennzahl ist die sogenannte Nitrierkennzahl K_{N}, die sich als Quotient aus dem Partialdruck von Ammoniak und der 1,5-fachen Potenz des Partialdrucks von Wasserstoff ergibt. Die Nitrierkennzahl K_{N} steht in einem direkten Bezug zu der Stickstoffaktivität und läßt anhand von Tabellenwerken, beispielsweise dem "Lehrer-Diagramm", den Existenzbereich eines gewünschten Nitrids in Abhängigkeit der Temperatur erkennen. Neben der Zusammensetzung des Nitrids können aus dem Wert der Nitrierkennzahl K_{N} auch Rückschlüsse über die Geschwindigkeit des Aufbaus einer Nitridschicht gewonnen werden. Für die Praxis bedeutet dies, daß die Betriebsweise eines Wärmebehandlungsofens nach einer vorgegebenen Nitrierkennzahl gesteuert wird. Da die Behandlungstemperatur während eines Nitrierungsprozesses in der Regel zwischen 500°C und 600°C liegt, ist die Ammoniakkonzentration aufgrund der bei diesen Temperaturen niedrigen Zersetzungsgenetik wesentlich höher als für eine Gleichgewichtsreaktion benötigt. Dies erfordert, daß ständig ein Teil der Ofenatmosphäre ausgetauscht bzw. mit frischem Ammoniak aufgefrischt werden muß, um eine gewünschte Nitrierkennzahl einzuhalten. Eine möglichst rationelle Arbeitsweise eines Wärmebehandlungsofens bedingt daher die exakte Kenntnis des Verhältnisses von Wasserstoff zu Ammoniak.

Im Stand der Technik ist es bekannt, für die Ermittlung der Nitrierkennzahl Gasanalyseverfahren zu verwenden, die auf Wärmeleitfähigkeits- oder Infrarot-Absorptionsprinzipien beruhen. Der Gehalt von Wasserstoff wird dabei über die Messung der Wärmeleitfähigkeit ermittelt, während der Gehalt von Ammoniak nach der Infrarot-Absorptionsmethode bestimmt wird. Als Nachteil hierbei erweist sich, daß zum einen die Verfahren verhältnismäßig teuer und aufwendig sind, und zum anderen, daß sie durch Undichtigkeits- und Kondensationsprobleme in den Zuleitungen zur Analyse nicht betriebssicher sind.

Im Stand der Technik ist es weiterhin bekannt, die Nitrierkennzahl indirekt über die Messung des Sauerstoffpartialdruckes mit einer Feststoff-Elektrolyt-Sonde zu ermitteln. Ursächlich hierfür ist, daß Sauerstoff mit Wasserstoff, Wasserdampf und etwaigen anderen Reaktionspartnern im Gleichgewicht steht. Wird ein derartiges Gasgemisch einer Seite des Feststoff-Elektrolyts zugeführt, und der anderen Seite ein Gasgemisch, welches zuvor über eine hocherhitzte katalytische Zone geleitet wurde, um den Ammoniak in Stickstoff und Wasserstoff zu spalten, was zu einer entsprechenden Verschiebung des Gleichgewichts des Wasserstoffs gegenüber den anderen Reaktionspartnern führt, so gibt die somit erhaltene Zellenspannung den Spaltgrad des Ammoniaks an. Nachteilig hierbei ist, daß diese Sonde nur in O₂-haltigen Atmosphären funktioniert und erhebliche Querempfindlichkeit zu Kohlenwasserstoffen besitzt.

US-A-5,668,301 offenbart einen Sensor zur Bestimmung von Wasserstoff, der aus einem mit einer Titan-Palladium-Legierung beschichteten Substrat verbunden mit einer elektrischen Schaltung besteht. US-A-4,882,032 offenbart einen elektrochemischen Sensor zur Bestimmung des Gehalts von Wasserstoff in geschmolzenem Aluminium in dem die Arbeitselektrode eine permselektive katalytische metallische Membran aufweist. US-A-5,088,315 offenbart eine Methode zur Bestimmung der Reinheit von einer aus zwei Komponenten einer binären Gasmischung mittels Gaspartialdruckmessungen.

Der Erfindung liegt die **Aufgabe** zugrunde, ein Verfahren der eingangs genannten Art dahingehend weiterzubilden, daß sich auf schnellansprechende, einfache und kostengünstige Weise der Gehalt von Wasserstoff sicher reproduzierbar auch bei hohen Temperaturen oberhalb 500°C bestimmen läßt und eine direkte Ermittlung von Prozeßkennzahlen ermöglicht wird.

Die Aufgabe ist erfindungsgemäß dadurch **gelöst,** daß Wasserstoff mittels einer permselektiven Membran aus dem Gasgemisch abgeschieden wird, daß dabei ein die Membran umströmender Gasstrom erzeugt wird, daß der Partialdruck des abgeschiedenen Wasserstoffs gemessen und als Maß für den Gehalt von Wasserstoff im Gasstrom herangezogen wird, und daß die Strömungsgeschwindigkeit des Gasstroms entlang der Oberfläche der Membran von einer eine katalytische Bildung zusätzlichen Wasserstoffs im Gasstrom verhindernden Höhe gewählt wird.

Durch ein solches Verfahren läßt sich auf einfache und kostengünstige Weise eine direkte Bestimmung der Nitrierkennzahl erreichen. Der Druck des durch die Membran abgeschiedenen Wasserstoffs steht dabei in einem nahen Gleichgewicht mit dem Wasserstoffpartialdruck des Gasstroms auf der anderen Seite der Membran, so daß dieser als ausreichend genaues Maß für den Gehalt von Wasserstoff im Gasstrom herangezogen werden kann.

Dem erfindungsgemäßen Verfahren liegt die überraschende Erkenntnis zugrunde, daß eine katalytische Wirkung der Membran auch bei hohen Temperaturen oberhalb von 500°C ausbleibt, wenn ein die Membran umströmender Gasstrom erzeugt wird, der durch eine vorgegebene Strömungsgeschwindigkeit definiert ist. Gemäß einem weiteren vorteilhaften Merkmal der Erfindung wird die Temperatur der Membran auf Werte im Bereich von 300 bis 1000°C, bei ammoniakhaltigen Gasatmosphären vorzugsweise im Bereich von 400 bis 650°C, eingestellt, um in Abhängigkeit des verwendeten Materials für die Membran die jeweilige spezifische Genetik der katalytischen Abspaltungsreaktion zu berücksichtigen. Von besonderem Vorteil ist es, wenn bei ammoniakhaltigen Gasatmosphären die Strömungsgeschwindigkeit des Gasstroms entlang der Oberfläche der Membran mindestens 140 cm/min beträgt, um eine katalytische Wirkung der Membran auszuschließen. Vorzugsweise wird als Material für die Membran eine Edelmetall-Legierung eingesetzt, welche dadurch mit Temperaturen bis über 1.000°C belastet werden kann.

Gemäß einem weiteren Merkmal der Erfindung wird zur Messung des gebundenen Wasserstoffanteils der Gasstrom vor Permeation des Wasserstoffs durch die Membran mit einem Katalysator in Berührung gebracht, der eine Spaltung von wasserstoffhaltigen Verbindungen im Gasstrom unter Bildung zusätzlichen Wasserstoffs hervorruft, und die Veränderung des dann gemessenen Wasserstoffpartialdruckes als Maß für den Gehalt an wasserstoffhaltigen Verbindungen im Gasstrom herangezogen. Ist beispielsweise die wasserstoffhaltige Verbindung im Gasstrom Ammoniak (NH₃), läßt sich die Nitrierkennzahl direkt bestimmen, indem die Differenz des Wasserstoffpartialdruckes des infolge der katalytischen Aufspaltung des Ammoniaks mit Wasserstoff angereicherten Gasgemisches und eines, beispielsweise zuvor gemessenen Wasserstoffpartialdruckes des noch unzersetzten Gasgemisches als Maß für den Gehalt von Ammoniak im Gasstrom herangezogen und in ein Verhältnis mit dem Gehalt von Wasserstoff im Gasstrom gesetzt wird.

Gemäß einem weiteren Merkmal der Erfindung wird durch Veränderungen der Temperatur und/oder der Strömungsgeschwindigkeit des Gasstroms die Oberfläche der Membran zeitweise katalytisch wirkend gemacht, um die Bestimmung des Gehalts einer wasserstoffhaltigen Verbindung zu ermitteln. Zweckmäßigerweise wird der Wasserstoffpartialdruck über eine erste und zweite Membran gemessen, wobei an einer der beiden Membranen der Gasstrom mit Wasserstoff infolge der katalytischen Spaltung von wasserstoffhaltigen Verbindungen angereichert ist, so daß eine gleichzeitige Messung des Gehalts von Wasserstoff und wasserstoffhaltigen Verbindungen möglich ist, was eine kontinuierliche Bestimmung beispielsweise der Nitrierkennzahl gestattet.

Eine Vorrichtung zur Durchführung des Verfahrens weist vorzugsweise eine von einem Rohr konzentrisch umgebene, kapillarenförmige, permselektive Membran auf, die in axialer Richtung in einem zwischen ihrer äußeren Mantelfläche und der Innenfläche des Rohres gebildeten Ringspalt von einem Gasstrom umströmt ist, wobei der Innenraum der Membran an einen Drucksensor anschließbar ist. Eine solchermaßen ausgestaltete Vorrichtung ermöglicht eine einfache und kostengünstige, das heißt besonders praxisgerechte Bestimmung des Gehalts von freiem oder in wasserstoffhaltigen Verbindungen gebundenem Wasserstoff nach dem erfindungsgemäßen Verfahren. Der zwischen Membran und Rohr ausgebildete Ringspalt erlaubt eine Zwangsführung des Gastroms entlang der Oberfläche der Membran derart, daß eine katalytische Wirkung dieser ausbleibt. Vorteilhafterweise ist der Innenraum der Membran evakuierbar, um sicherzustellen, daß der von dem Drucksensor erfaßte Druck ausschließlich von mittels Permeation in den Innenraum gelangten Wasserstoff herrührt.

In einer bevorzugten Ausführungsform ist die Membran an eine Hohlkammer angeschlossen, welche mit dem Drucksensor und über ein Absperrventil mit einer Vakuumpumpe verbunden ist, so daß eine besonders einfache und kompakte Bauweise erzielt wird. Zweckmäßigerweise ist die Membran aus einer Palladium-Silber-Legierung gefertigt, um einerseits für Wasserstoff permeabel zu sein und andererseits hohen Temperaturen auch oberhalb von 1.000°C Stand zu halten. Zweckmäßig ist ferner, wenn zumindest im Bereich des Eintritts des Gasstroms in den zwischen Membran und Rohr gebildeten Ringspalt ein Heizelement angeordnet ist, so daß Schwankungen der für die Permeation erforderlichen Temperatur ausgeglichen werden können, wodurch eine gleichmäßige Strömungsgeschwindigkeit des Gasstroms aufrecht erhalten werden kann.

Um auf einfache und kostengünstige Weise eine Zwangsströmung des Gasstroms zu erzielen, ist gemäß einem weiteren vorteilhaften Merkmal der Erfindung die Strömungsgeschwindigkeit des Gasstroms durch den Ringspalt über eine an das Rohr angeschlossene Pumpe und/oder ein Drosselventil steuerbar.

Gemäß einer vorteilhaften Ausgestaltung der Erfindung ist ein Katalysator stromaufwärts der Membran angeordnet, so daß eine Spaltung von bestimmten wasserstoffhaltigen Verbindungen im Gasstrom unter Bildung zusätzlichen Wasserstoffs gewährleistet ist. Von Vorteil ist es, wenn hierzu der Katalysator über ein Ventil und eine Bypassleitung wahlweise der Membran vorschaltbar ist. Auf diese weise kann nacheinander der Gehalt von Wasserstoff und von bestimmten wasserstoffhaltigen Verbindungen im Gasstrom ermittelt werden.

Zweckmäßigerweise ist der durch den Katalysator geleitete Gasstrom mittels eines Heizelementes auf Temperaturen über 1.000°C aufheizbar, um eine Spaltung, beispielsweise von Ammoniak, unter Bildung von Wasserstoff sicherzustellen. Zu diesem Zweck weist der Katalysator vorzugsweise eine reaktive Oberfläche aus einer Schüttung von katalytisch wirkenden Nickel- oder Edelmetallstücken auf.

Gemäß einer weiteren vorteilhaften Ausgestaltung der Erfindung sind zwei Membranen parallel zueinander geschaltet, wobei einer der beiden Membranen der Katalysator vorgeschaltet ist. Dieses bietet die Möglichkeit der gleichzeitigen Messung des Gehalts von Wasserstoff und wasserstoffhaltigen Verbindungen, so daß eine kontinuierliche Ermittlung beispielsweise der Nitrierkennzahl ermöglicht wird. Gemäß einer alternativen Ausgestaltung sind zwei Membranen in Serie geschaltet, wobei der weiter stromabwärts angeordneten Membran der Katalysator vorgeschaltet ist. Vorteilhaft hierbei ist, daß beide Membranen mit ein und demselben Meßgas versorgt werden, wodurch sich eine sehr kompakte Bauweise erreichen läßt.

Schließlich wird vorgeschlagen, daß die Vorrichtung direkt an einen Wärmebehandlungsofen angeflanscht ist, um eine ständige Bestimmung des Gehalts von freiem und/oder gebundenem Wasserstoff in der Gasatmosphäre des Wärmebehandlungsofens zu ermöglichen.

Weitere Merkmale und Vorteile des Gegenstandes der Erfindung ergeben sich aus der nachfolgenden Beschreibung bevorzugter Ausführungsbeispiele, mit denen das erfindungsgemäße Verfahren realisiert werden kann. In der zugehörigen Zeichnung zeigen:
- Fig. 1: eine Vorrichtung zur quantitativen Bestimmung des Gehaltes von reinem und/oder gebundenem Wasserstoff in Form einer direkt an einem Ofen befestigten Sonde in einer schematischen Darstellung;
- Fig. 2: eine alternative Ausführungsform gemäß Fig. 1 in Form eines autonomen Meßgerätes in einer schematischen Darstellung;
- Fig. 3: eine weitere alternative Ausführungsform einer Vorrichtung gemäß Fig. 1 oder 2 in einer schematischen Darstellung und
- Fig. 4: eine Vorrichtung gemäß Fig. 1 in Form einer an einer Wand eines Ofens montierbaren Sonde in einem Querschnitt.

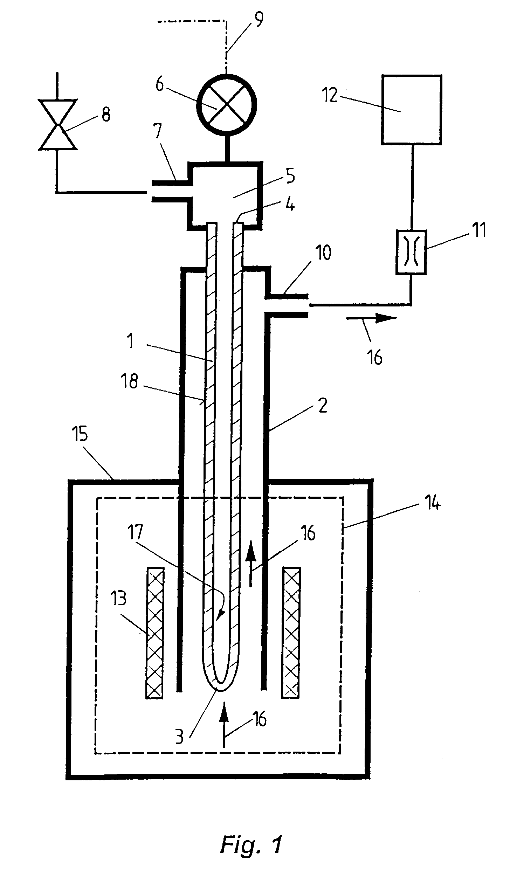

Die in Fig. 1 in schematischer Weise gezeigte Vorrichtung zur quantitativen Bestimmung des Gehaltes von reinem und/oder gebundenem Wasserstoff weist eine kapillarenförmige, permselektive Membran 1 auf, die von einem Rohr 2 konzentrisch umgeben ist. Die aus einer Palladium-Silber-Legierung bestehende Membran 1 ist an einem stirnseitigen Ende 3 geschlossen und am gegenüberliegenden Ende 4 offen ausgebildet. Eine ein geringes Volumen aufweisende Hohlkammer 5 schließt sich an das offen ausgebildete Ende 4 der Membran 1 an und ist mit einem Drucksensor 6 sowie über einen Anschluß 7 und ein Absperrventil 8 mit einer nicht dargestellten Vakuumpumpe 9 verbunden. Der Drucksensor 6 ist als piezoelektrischer Sensor ausgebildet und steht über eine Signalleitung 9 mit einem ebenfalls nicht dargestellten Meß- und/oder Regelsystem in Verbindung.

Das die Membran 1 konzentrisch umgebende Rohr 2 ist im Bereich des Endes 3 der Membran 1 stirnseitig offen, während an der gegenüberliegenden Stirnseite 9 die Membran 1 gasdicht herausgeführt wird. Das Rohr 2 ist weiterhin mit einer Öffnung 10 versehen, an die ein Drosselventil 11 und/oder eine Pumpe 12 angeschlossen ist. Ein Heizelement 13 umgibt das Rohr 2 im Bereich des stirnseitig offenen Endes.

Die Membran 1 ragt zusammen mit dem Rohr 2 in die heiße Zone 14 eines Nitrierofens 15 hinein. Zu diesem Zweck kann das Rohr 2 mittels eines nicht dargestellten Flansches an die Wandung des Nitrierofens 15 befestigt sein. Zur Bestimmung des Gehaltes von Wasserstoff oder wasserstoffhaltigen Verbindungen in der Gasatmosphäre des Nitrierofens 15 wird durch die Pumpe 12 ein Gasstrom 16 aus der heißen Zone 50 herausgepumpt, dessen Strömungsgeschwindigkeit durch das Drosselventil 11 steuerbar ist. Der Gasstrom 16 umströmt die Membran 1, wodurch Wasserstoff durch Permeation in einen Innenraum 17 der Membran 1 dringt. Der Innenraum 17 der Membran 1 ist über das Ventil 8 und die nicht dargestellte Vakuumpumpe evakuierbar, 50 daß sich im Innenraum 17 ein Druck aufbaut, der ausschließlich von dem Wasserstoff herrührt und über den Drucksensor 6 erfaßt wird. Der mit dem Drucksensor 6 gemessene Wasserstoffdruck steht in einem nahen Gleichgewicht mit dem Wasserstoffpartialdruck des Gasstromes 16 und kann daher als Maß für den Gehalt an Wasserstoff der Gasatmosphäre des Nitrierofens 15 herangezogen werden.

Eine katalytische Wirkung der aus einer Palladium-Silber-Legierung bestehenden Membran 1 auf den Gasstrom 16 bleibt infolge der Strömungsgeschwindigkeit des Gasstromes 16 aus. Messungen hierzu ergaben, daß selbst bei Temperaturen bis 630°C ein Volumenstrom von 15 N cm³/min ausreichend ist. Um auch eine katalytische Wirkung auf den Gasstrom 16 seitens des Rohrs 2 auszuschließen, besteht dieses aus einem Material, welches keine katalytische Wirkung ausübt, beispielsweise Quarz. Durch das Heizelement 13 läßt sich der Gasstrom 16 auf eine für die Permeation erforderliche Temperatur aufheizen, wenn die Temperatur der heißen Zone 14 zu gering ist.

Für die Bestimmung des Gehaltes von wasserstoffhaltigen Verbindungen im Gasstrom, wie beispielsweise das für Nitrierprozesse häufig verwendete Ammoniak (NH₃), ist es erforderlich, diese zu spalten, so daß sich aufgrund des dann im Gasstrom 16 enthaltenen Wasserstoffs ein erhöhter Wasserstoffpartialdruck messen läßt, der im Vergleich mit dem Wasserstoffpartialdruck eines unzersetzten Gasstroms 16 als Maß für den Gehalt der wasserstoffhaltigen Verbindungen im unzersetzten Gasstrom 16 herangezogen werden kann. Die Spaltung wasserstoffhaltiger Verbindungen im Gasstrom 16 läßt sich durch Veränderung der Strömungsgeschwindigkeit und/oder der Temperatur des Gasstromes 16 erreichen, indem diese derart verändert werden, daß von der Oberfläche 18 der Membran 1 eine katalytische Wirkung ausgeht. Zu diesem Zweck kann sowohl die Förderleistung der Pumpe 12 erniedrigt werden, als auch die Temperatur des Gasstromes 16 über das Heizelement 13 auf Temperaturen von beispielsweise über 1000°C erhöht werden.

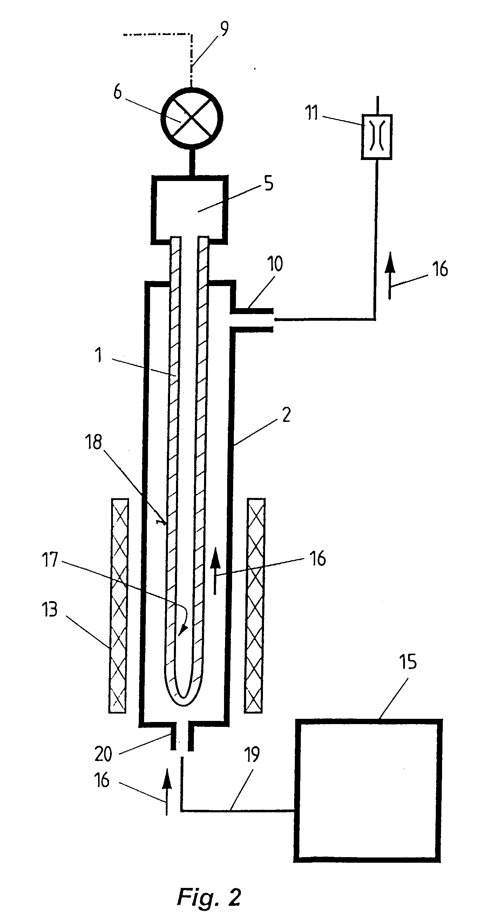

Während Fig. 1 eine direkt in die heiße Zone 14 des Nitrierofens 15 hineinreichende Anordnung zeigt, ist in Fig. 2 eine alternative Ausführungsform dargestellt, bei der der Gasstrom 16 aus dem Nitrierofen 16 über eine Gasleitung 19 einem stirnseitigen Anschluß 20 des Rohrs 2 zugeführt wird. Die als autonomes Meßgerät ausführbare Anordnung wird ohne eine Pumpe 12 betrieben, wofür verantwortlich ist, daß der Nitriervorgang im Nitrierofen 15 bei einem Überdruck erfolgt, so daß es ausreichend ist, den von alleine sich einstellenden Gasstrom 16 über das Ventil 11, das beispielsweise als präzises Nadelventil ausgeführt sein kann, zu steuern. Auch in dieser Ausführungsform ist es möglich, sowohl den Gehalt von Wasserstoff als auch von wasserstoffhaltigen Verbindungen im Gasstrom 16 zu messen, um dadurch die Nitrierkonstante K_{N} zu ermitteln. Versuchsmessungen hierzu ergaben, daß bei einer Ofentemperatur von 400°C und einem eingestellten Volumenstrom von 5 N cm³/min sich der Gehalt von reinem Wasserstoff im Gasstrom 16 ermitteln ließ. Bei Steigerung der Temperatur auf 900°C hingegen lag ein erhöhter Wasserstoffpartialdruck vor, der aus zusätzlichem Wasserstoff infolge einer Spaltung von Ammoniak herrührte. Durch die Differenz der Druckwerte bei 400°C und 900°C ließ sich der Anteil des Ammoniaks im Gasstrom 16 und dadurch der Wert der Nitrierkonstante K_{N} in einem aufheiz- und abkühlbedingten Zeitraum von lediglich 15 Minuten ermitteln.

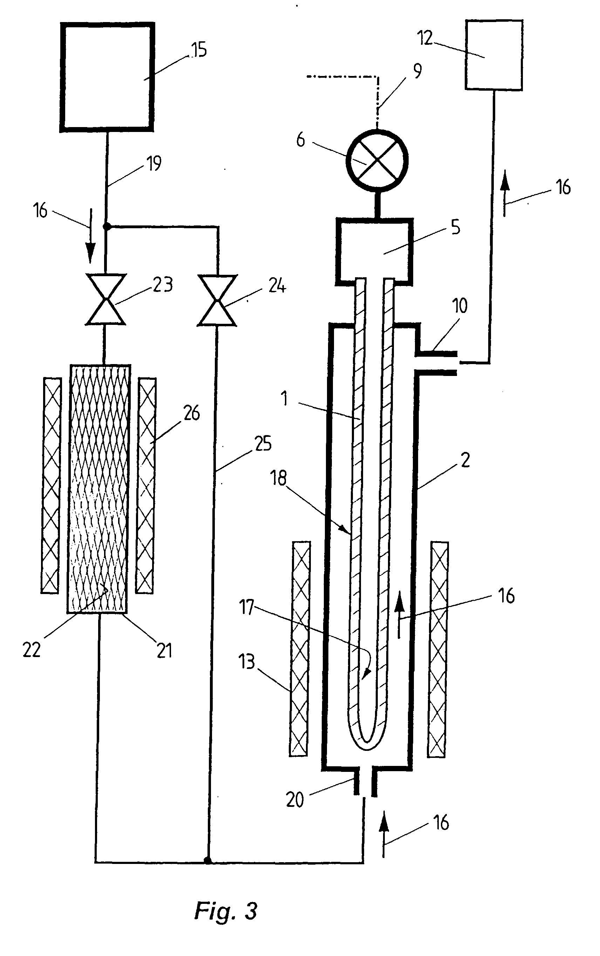

In Fig. 3 ist eine weitere alternative Ausführungsform dargestellt. Im Unterschied zu Fig. 2 ist dabei zwischen Nitrierofen 15 und Rohr 2 eine Katalysator 21 geschaltet, der eine reaktive Oberfläche 22 aufweist, die aus einer katalytisch wirkenden Nickelbeschichtung besteht. Der Katalysator 21 ist über Ventile 23, 24 und eine Bypassleitung 25 wahlweise dem Rohr 2 vorschaltbar. Der Gasstrom 16 wird wieder über eine Pumpe 11 erzeugt, welche hierfür mit einer exakten und variabel einstellbaren Förderleistung ausgebildet ist. Der in den Innenraum 17 der Membran 1 eindringende Wasserstoff wird über die Hohlkammer 5 dem Drucksensor 6 zugeführt, wobei dieser nur die Rolle eines Übergangsstückes zukommt. Die Evakuierung des Innenraums 17 erfolgt - wie auch bei der Ausführungsform gemäß Fig. 2 - über eine nicht dargestellte Vakuumpumpe. Der Katalysator 21 ist von einem Heizelement 26 umgeben, durch welches Temperaturen von über 1000°C zu erzeugen sind. Durch die alternative Anordnung gemäß Fig. 3 ist es möglich, den Gehalt von Wasserstoff und wasserstoffhaltigen Verbindungen im Gasstrom 16 in einer sehr kurzen Zeit zu ermitteln, indem der Gasstrom 16 zuerst über die Bypassleitung 25 geführt wird, wodurch sich der Gehalt an reinem Wasserstoff im unzersetzten Gasstrom 16 ermitteln läßt, und im Anschluß daran über den Katalysator 21 strömt, so daß eine Spaltung beispielsweise von Ammoniak stattfindet und der Drucksensor 6 den Druck eines mit Wasserstoff angereicherten Gasstroms 16 mißt. Die Differenzbildung der beiden Wasserstoffdrücke erlaubt wiederum die Berechnung des Gehaltes von Ammoniak im Gasstrom 16.

Für eine kontinuierliche Messung von Wasserstoff und wasserstoffhaltigen Verbindungen kann die Ausführungsform gemäß Fig. 2 mit der Ausführungsform gemäß Fig. 3 kombiniert werden, indem diese parallel oder hintereinandergeschaltet mit demselben Gasstrom 16 versorgt werden. Die Hintereinanderschaltung ist derart, daß der Gasstrom 16 nach dem Durchströmen eines ersten Rohres 2, wobei der Wasserstoffpartialdruck eines unzersetzten Gasstroms 16 gemessen wird, über den Katalysator 21, welcher eine Spaltung wasserstoffhaltiger Verbindungen bewirkt, einem zweiten Rohr 2 zugeführt wird, wobei dann der Wasserstoffpartialdruck des mit Wasserstoff infolge der Spaltung angereicherten Gasstroms 16 ermittelt wird. Um eine kompakte Bauweise zu erzielen, ist es möglich, die Rohre 2 sowie den Katalysator 21 innerhalb ein und desselben Heizelementes 13 anzuordnen.

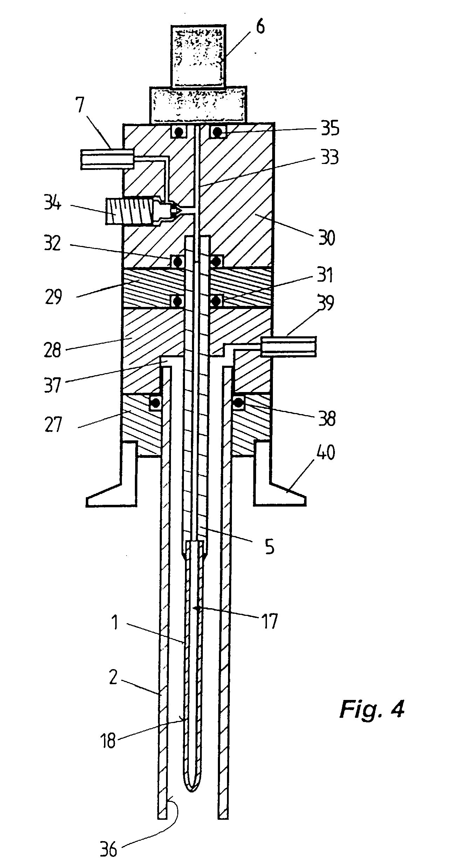

Fig. 4 zeigt eine konkrete Ausgestaltungsform einer Wasserstoffsonde zur Messung des Gehaltes von Wasserstoff in der Gasatmosphäre von Wärmebehandlungsöfen. Die aus einer Palladium-Silber-Legierung bestehende Membran 1 ist an die als Nickelrohr ausgebildete Hohlkammer 5 angelötet, welche sich durch Blockelemente 27, 28, 29 und 30 erstreckt und mittels Ringdichtungen 31, 32 gasdicht abgedichtet ist. Die rohrförmige Hohlkammer 5 mündet in einer Bohrung 33 des Blockelementes 30, die mit dem Drucksensor 6 und über eine Ventilschraube 34 mit dem Anschluß 7 verbunden ist. Der Drucksensor 6 ist über eine Ringdichtung 35 gasdicht an das Blockelement 30 angeschlossen.

Das Rohr 2 umgibt konzentrisch die Membran 1 und teilweise die rohrförmige Hohlkammer 5, wobei am stirnseitigen Ende 3 der Membran 1 das Rohr 2 diese in axialer Richtung in einer gewissen Länge überragt. Das aus Stahl gefertigte Rohr 2 ist an seiner Innenfläche mit einer Keramik emailliert, um eine katalytische Wirkung auf den Gasstrom 16 auszuschließen. Das Rohr 2 wird in einer zentralen Bohrung 37 in den Blockelementen 27 und 28 gehalten und ist mittels einer Ringdichtung 38 nach außen hin abgedichtet. Ein in den zwischen der äußeren Mantelfläche 18 der Membran 1 und der Innenfläche 36 des Rohres 2 gebildeten Ringspalt einströmender Gasstrom 16 gelangt über einen Abströmanschluß 39 zu dem Drosselventil 11 und/oder der Pumpe 12. Über einen Flansch 40 ist die Wasserstoffsonde direkt an eine Wandung eines Wärmebehandlungsofens so befestigbar, daß die Membran 1 und das sie umgebende Rohr 2 in die heiße Zone des Wärmebehandlungsofens hineinragt. Der Innenraum 17 der Membran 1 und der sich anschließenden rohrförmigen Hohlkammer 5 ist über eine an den Anschluß 7 anschließbare Vakuumpumpe evakuierbar. Der Evakuierungsprozeß kann durch Einleiten von Wasserstoff an dem Abströmanschluß 39, welcher dann im Gegenstrom am stirnseitig offenen Ende des Rohres 2 wieder austritt, unterstützt werden, indem der dann in den Innenraum 17 eindiffundierende Wasserstoff als Spülgas dient. In gleicher Weise können anstelle von Wasserstoff auch Kalibriergasgemische mit bekannten Anteilen an Wasserstoff und wasserstoffhaltigen Verbindungen eingeleitet werden, so daß eine einfache Kontrolle der Meßgenauigkeit erzielt wird.

Mit dem anhand der oben erwähnten Ausführungsbeispiele beschriebenen Verfahren ist auf einfache und kostengünstige Weise der Gehalt von Wasserstoff und wahlweise auch von wasserstoffhaltigen Verbindungen eines Gasgemisches zu ermitteln. Dies ist wichtig für die Bestimmung von Kennzahlen, wie beispielsweise der Nitrierkennzahl K_{N}, die für die Prozeßsteuerung von Wärmebehandlungsverfahren notwendig sind. Mit dem zuvor beschriebenen Verfahren ist es möglich, auch bei hohen Temperaturen von mehr als 500°C den Gehalt von reinem Wasserstoff zu ermitteln, ohne daß eine Verfälschung des Ergebnisses durch eine katalytische Aufspaltung von wasserstoffhaltigen Verbindungen stattfindet.

Ein solches Verfahren eignet sich zur Analyse verschiedenster Prozeßgase und kann durch die Verwendung eines geeigneten Materials für die Membran 1 auch zur Bestimmung von anderen Stoffen als Wasserstoff und daraus abgeleiteten Kennzahlen auf einfache und schnelle Weise modifiziert werden.

### Bezugszeichenliste

- 1: Membran
- 2: Rohr
- 3: stirnseitiges Ende
- 4: stirnseitiges Ende
- 5: Hohlkammer
- 6: Drucksensor
- 7: Anschluß
- 8: Ventil
- 9: Signalleitung
- 10: Öffnung
- 11: Drosselventil
- 12: Pumpe
- 13: Heizelement
- 14: heiße Zone
- 15: Nitrierofen
- 16: Gasstrom
- 17: Innenraum
- 18: Oberfläche
- 19: Gasleitung
- 20: Anschluß
- 21: Katalysator
- 22: Oberfläche
- 23: Ventil
- 24: Ventil
- 25: Bypassleitung
- 26: Heizelement
- 27: Blockelement
- 28: Blockelement
- 29: Blockelement
- 30: Blockelement
- 31: Ringdichtung
- 32: Ringdichtung
- 33: Bohrung
- 34: Ventilschraube
- 35: Ringdichtung
- 36: Innenfläche
- 37: Bohrung
- 38: Ringdichtung
- 39: Abströmanschluß
- 40: Flansch

## Patentansprüche

1. Verfahren zur quantitativen Bestimmung des Gehalts von reinem und/oder gebundenem Wasserstoff in wasserstoffhaltigen Gasgemischen, vorzugsweise in ammoniak- oder kohlenwasserstoffhaltigen Gasatmosphären von Wärmebehandlungsöfen,
**dadurch gekennzeichnet,**
daß Wasserstoff mittels einer permselektiven Membran (1) aus dem Gasgemisch abgeschieden wird, daß dabei ein die Membran (1) umströmender Gasstrom (16) erzeugt wird, daß der Partialdruck des abgeschiedenen Wasserstoffs gemessen und als Maß für den Gehalt von Wasserstoff im Gasstrom (16) herangezogen wird, und daß die Strömungsgeschwindigkeit des Gasstroms (16) entlang der Oberfläche (18) der Membran (1) von einer eine katalytische Bildung zusätzlichen Wasserstoffs im Gasstrom (16) verhindernden Höhe gewählt wird.

2. Verfahren nach Anspruch 1, dadurch gekennzeichnet, daß die Temperatur der Membran (1) auf Werte im Bereich von 300 bis 1000°C, bei ammoniakhaltigen Gasatmosphären vorzugsweise im Bereich von 400 bis 650°C, eingestellt wird.

3. Verfahren nach Anspruch 2, dadurch gekennzeichnet, daß bei ammoniakhaltigen Gasatmosphären die Strömungsgeschwindigkeit des Gasstroms (16) entlang der Oberfläche (18) der Membran (1) mindestens 140 cm/min beträgt.

4. Verfahren nach einem der Ansprüche 1 bis 3, dadurch gekennzeichnet, daß als Material für die Membran (1) eine Edelmetall-Legierung eingesetzt wird.

5. Verfahren nach einem der Ansprüche 1 bis 4, dadurch gekennzeichnet, daß zur Messung des gebundenen Wasserstoffanteils der Gasstrom (16) vor Permeation des Wasserstoffs durch die Membran (1) mit einem Katalysator (21) in Berührung gebracht wird, der eine Spaltung von wasserstoffhaltigen Verbindungen im Gasstrom (16) unter Bildung zusätzlichen Wasserstoffs hervorruft, und daß die Veränderung des dann gemessenen Wasserpartialdruckes als Maß für den Gehalt an wasserstoffhaltigen Verbindungen im Gasstrom (16) herangezogen wird.

6. Verfahren nach Anspruch 5, dadurch gekennzeichnet, daß durch Veränderungen der Temperatur und/oder der Strömungsgeschwindigkeit des Gasstromes (16) die Oberfläche (18) der Membran (1) zeitweise katalytisch wirkend gemacht wird.

7. Verfahren nach einem der Ansprüche 5 oder 6, dadurch gekennzeichnet, daß der Wasserstoffpartialdruck über eine erste und zweite Membran (1) gemessen wird, wobei an einer der beiden Membranen (1) der Gasstrom (16) mit Wasserstoff infolge der katalytischen Spaltung von wasserstoffhaltigen Verbindungen angereichert ist.

8. Vorrichtung zur Durchführung des Verfahren nach einem der Ansprüche 1 bis 7, gekennzeichnet durch eine von einem Rohr (2) konzentrisch umgebene, kapillarenförmige, permselektive Membran (1), die in axialer Richtung in einem zwischen ihrer äußeren Mantelfläche (18) und der Innenfläche (36) des Rohres (2) gebildeten Ringspalt von einem Gasstrom (16) umströmt ist, wobei der Innenraum (17) der Membran (1) an einen Drucksensor (6) anschließbar ist.

9. Vorrichtung nach Anspruch 8, dadurch gekennzeichnet, daß der Innenraum (17) der Membran (1) evakuierbar ist.

10. Vorrichtung nach Anspruch 8 oder 9, dadurch gekennzeichnet, daß die Membran (1) an eine Hohlkammer (5) angeschlossen ist, welche mit dem Drucksensor (6) und über ein Absperrventil (8) mit einer Vakuumpumpe verbunden ist.

11. Vorrichtung nach einem der Ansprüche 8 bis 10, gekennzeichnet durch eine Membran (1) aus einer Palladium-Silber-Legierung.

12. Vorrichtung nach einem der Ansprüche 8 bis 11, dadurch gekennzeichnet, daß zumindest im Bereich des Eintritts des Gasstromes (16) in dem zwischen Membran (1) und Rohr (2) gebildeten Ringspalt ein Heizelement (13) angeordnet ist.

13. Vorrichtung nach einem der Ansprüche 8 bis 12, dadurch gekennzeichnet, daß die Strömungsgeschwindigkeit des Gasstromes (16) durch den Ringspalt über eine an das Rohr angeschlossene Pumpe (12) und/oder ein Drosselventil (11) steuerbar ist.

14. Vorrichtung nach einem der Ansprüche 8 bis 13, dadurch gekennzeichnet, daß ein Katalysator (21) stromaufwärts der Membran angeordnet ist.

15. Vorrichtung nach Anspruch 14, dadurch gekennzeichnet, daß der Katalysator (21) über ein Ventil (23, 24) und eine Bypassleitung (25) wahlweise der Membran (1) vorschaltbar ist.

16. Vorrichtung nach Anspruch 14 oder 15, dadurch gekennzeichnet, daß der durch den Katalysator (21) geleitete Gasstrom (16) mittels eines Heizelementes (26) auf Temperaturen über 1000°C aufheizbar ist.

17. Vorrichtung nach einem der Ansprüche 14 bis 16, dadurch gekennzeichnet, daß der Katalysator (21) eine reaktive Oberfläche aus einer Schüttung von katalytisch wirkenden Nickel- oder Edelmetallstücken aufweist.

18. Vorrichtung nach einem der Ansprüche 8 bis 17, gekennzeichnet durch die parallele Anordnung zweier Membranen (1), wobei einer der beiden Membranen der Katalysator (21) vorgeschaltet ist.

19. Vorrichtung nach einem der Ansprüche 8 bis 17, gekennzeichnet durch die serielle Anordnung zweier Membranen (1), wobei der weiter stromabwärts angeordneten Membran (1) der Katalysator (21) vorgeschaltet ist.

20. Vorrichtung nach einem der vorhergehenden Ansprüche, gekennzeichnet durch ein direktes Anflanschen an einen Wärmebehandlungsofen (15).

## Claims

1. Method for the quantitative determination of the content of pure and/or combined hydrogen in gas mixtures containing hydrogen, preferably in gaseous atmospheres containing ammonia or hydrocarbons of heat treatment furnaces, characterised in that hydrogen is separated from the gas mixture by means of a permselective membrane (1), that a gas stream (16) flowing around the membrane (1) is then produced, that the partial pressure of the separated hydrogen is measured and used as a measure of the content of hydrogen in the gas stream (16), and that the flow speed of the gas stream (16) along the surface (18) of the membrane (1) is selected from a level preventing the catalytic generation of additional hydrogen in the gas stream (16).

2. Method according to Claim 1, characterised in that the temperature of the membrane (1) is set to values in the range from 300 to 1000°C, preferably in the range from 400 to 650°C for gaseous atmospheres containing ammonia.

3. Method according to Claim 2, characterised in that the flow speed of the gas stream (16) along the surface (18) of the membrane (1) is at least 140 cm/min. for gaseous atmospheres containing ammonia.

4. Method according to any one of Claims 1 to 3, characterised in that a noble metal alloy is used as the material for the membrane (1).

5. Method according to any one of Claims 1 to 4, characterised in that, in order to measure the proportion of combined hydrogen, prior to the hydrogen permeating through the membrane (1), the gas stream (16) is brought into contact with a catalyst (21) which causes decomposition of compounds in the gas stream (16) which contain hydrogen while generating additional hydrogen, and that the change in the partial pressure of the hydrogen which is then measured is used as a measure of the content of compounds in the gas stream (16) which contain hydrogen.

6. Method according to Claim 5, characterised in that the surface (18) of the membrane (1) is intermittently rendered catalytically active by changes in the temperature and/or the flow speed of the gas stream (16).

7. Method according to either of Claims 5 and 6, characterised in that the partial pressure of the hydrogen is measured via a first and second membrane (1), wherein the gas stream (16) is enriched with hydrogen at one of the two membranes (1) as a result of the catalytic decomposition of compounds which contain hydrogen.

8. Apparatus for carrying out the method according to any one of Claims 1 to 7, characterised by a capillary, permselective membrane (1), which is concentrically surrounded by a tube (2) and around which a gas stream (16) flows in the axial direction in an annular gap formed between its outer circumferential surface (18) and the inner surface (36) of the tube (2), wherein the interior space (17) of the membrane (1) can be connected to a pressure sensor (6).

9. Apparatus according to Claim 8, characterised in that the interior space (17) of the membrane (1) can be evacuated.

10. Apparatus according to Claim 8 or 9, characterised in that the membrane (1) is connected to a hollow chamber (5), which is connected to the pressure sensor (6) and via a shut-off valve (8) to a vacuum pump.

11. Apparatus according to any one of Claims 8 to 10, characterised by a membrane (1) of a palladium-silver alloy.

12. Apparatus according to any one of Claim 8 to 11, characterised in that a heating element (13) is arranged at least in the region where the gas stream (16) enters the annular gap formed between the membrane (1) and the tube (2).

13. Apparatus according to any one of Claim 8 to 12, characterised in that the flow speed of the gas stream (16) through the annular gap can be controlled via a pump (12), which is connected to the tube, and/or a throttle valve (11).

14. Apparatus according to any one of Claims 8 to 13, characterised in that a catalyst (21) is arranged upstream of the membrane.

15. Apparatus according to Claim 14, characterised in that the catalyst (21) may optionally be connected ahead of the membrane (1) via a valve (23, 24) and a bypass line (25).

16. Apparatus according to Claim 14 or 15, characterised in that the gas stream (16) which is routed through the catalyst (21) can be heated by means of a heating element (26) to temperatures in excess of 1000°C.

17. Apparatus according to any one of Claims 14 to 16, characterised in that the catalyst (21) comprises a reactive surface of a bed of catalytically active nickel or noble metal pieces.

18. Apparatus according to any one of Claims 8 to 17, characterised by a parallel arrangement of two membranes (1), wherein one of the two membranes is connected ahead of the catalyst (21).

19. Apparatus according to any one of Claims 8 to 17, characterised by a series arrangement of two membranes (1), wherein the membrane (1) arranged further downstream is connected ahead of the catalyst (21).

20. Apparatus according to any one of the preceding Claims, characterised by being directly flanged to a heat-treatment furnace (15).

## Revendications

1. Procédé destiné à la détermination quantitative de la teneur en hydrogène pur et/ou lié dans des mélanges gazeux contenant de l'hydrogène, de préférence dans des atmosphères gazeuses contenant de l'ammoniac ou des hydrocarbures de fours de traitement thermique,
caractérisé en ce que de l'hydrogène est séparé du mélange gazeux au moyen d'une membrane (1) à perméation sélective, en ce qu'il est en l'occurrence engendré un flux gazeux (16) circulant autour de la membrane (1), en ce que la pression partielle de l'hydrogène séparé est mesurée et utilisée comme critère pour la teneur en hydrogène dans le flux gazeux (16), et en ce que la vitesse de circulation du flux gazeux (16) le long de la surface (18) de la membrane (1) est choisie de manière à éviter une formation catalytique d'hydrogène supplémentaire dans le flux gazeux (16).

2. Procédé selon la revendication 1, caractérisé en ce que la température de la membrane (1) est réglée à des valeurs se situant dans la plage de 300 à 1 000 °C, dans le cas d'atmosphères gazeuses contenant de l'ammoniac, de préférence dans la plage de 400 à 650 °C.

3. Procédé selon la revendication 2, caractérisé en ce que, dans le cas d'atmosphères gazeuses contenant de l'ammoniac, la vitesse de circulation du flux gazeux (16) le long de la surface (18) de la membrane (1) est au moins de 140 cm/min.

4. Procédé selon l'une quelconque des revendications 1 à 3, caractérisé en ce que le matériau utilisé pour la membrane (1) est un alliage en métal précieux.

5. Procédé selon l'une quelconque des revendications 1 à 4, caractérisé en ce que pour la mesure de la teneur en hydrogène lié, le flux gazeux (16) est mis en contact avec un catalyseur (21) avant la perméation de l'hydrogène à travers la membrane (1), lequel provoque une décomposition de composés contenant de l'hydrogène dans le flux gazeux (16) en générant de l'hydrogène supplémentaire, et en ce que la modification de la pression partielle de l'hydrogène alors mesurée est utilisée en tant que critère pour la teneur en composés contenant de l'hydrogène dans le flux gazeux (16).

6. Procédé selon la revendication 5, caractérisé en ce que la surface (18) de la membrane (1) est rendue temporairement catalytiquement active par des variations de la température et/ou de la vitesse de circulation du flux gazeux (16).

7. Procédé selon l'une des revendications 5 ou 6, caractérisé en ce que la pression partielle de l'hydrogène est mesurée par l'intermédiaire d'une première et d'une deuxième membrane (1), le flux gazeux (16) étant enrichi en hydrogène sur l'une des deux membranes (1) suite à la décomposition catalytique de composés contenant de l'hydrogène.

8. Dispositif destiné à la mise en oeuvre du procédé selon l'une quelconque des revendications 1 à 7, caractérisé par une membrane capillaire (1) à perméation sélective, qui est entourée de façon concentrique par un tube (2), autour de laquelle un flux gazeux (16) circule dans la direction axiale dans une fente annulaire formée entre sa surface d'enveloppe extérieure (18) et la surface intérieure (36) du tube (2), l'espace intérieur (17) de la membrane (1) pouvant être raccordé à un détecteur de pression (6).

9. Dispositif selon la revendication 8, caractérisé en ce que l'espace intérieur (17) de la membrane (1) peut être mis sous vide.

10. Dispositif selon la revendication 8 ou 9, caractérisé en ce que la membrane (1) est raccordée à une chambre creuse (5) qui est reliée au détecteur de pression (6), et à une pompe à vide par l'intermédiaire d'une vanne d'arrêt (8).

11. Dispositif selon l'une quelconque des revendications 8 à 10, caractérisé par une membrane (1) en un alliage de palladium-argent.

12. Dispositif selon l'une quelconque des revendications 8 à 11, caractérisé en ce qu'un élément chauffant (13) est disposé dans la fente annulaire formée entre la membrane (1) et le tube (2), au moins dans la zone d'entrée du flux gazeux (16).

13. Dispositif selon l'une quelconque des revendications 8 à 12, caractérisé en ce que la vitesse de circulation du flux gazeux (16) à travers la fente annulaire peut être commandée par l'intermédiaire d'une pompe (12) et/ou d'une soupape d'étranglement (11) raccordées au tube.

14. Dispositif selon l'une quelconque des revendications 8 à 13, caractérisé en ce qu'un catalyseur (21) est disposé en amont de la membrane.

15. Dispositif selon la revendication 14, caractérisé en ce que le catalyseur (21) peut être branché facultativement en amont de la membrane (1) par l'intermédiaire d'une vanne (23, 24) et d'une conduite de dérivation (25).

16. Dispositif selon la revendication 14 ou 15, caractérisé en ce que le flux gazeux (16) dirigé à travers le catalyseur (21) peut être porté à des températures supérieures à 1 000 °C au moyen d'un élément chauffant (26).

17. Dispositif selon l'une quelconque des revendications 14 à 16, caractérisé en ce que le catalyseur (21) comporte une surface réactive constituée d'un revêtement de fragments de nickel ou de métal précieux à effet catalytique.

18. Dispositif selon l'une quelconque des revendications 8 à 17, caractérisé par la disposition parallèle de deux membranes (1), le catalyseur (21) étant branché en amont de l'une des deux membranes.

19. Dispositif selon l'une quelconque des revendications 8 à 17, caractérisé par la disposition en série de deux membranes (1), le catalyseur (21) étant branché en amont de la membrane (1) disposée plus en aval.

20. Dispositif selon l'une quelconque des revendications précédentes, caractérisé par un bridage direct sur un four de traitement thermique (15).
